Europäisches Patentamt

European Patent Office

Office européen des brevets

(19)

(11) Publication number: **0 244 992**
A2

(12) **EUROPEAN PATENT APPLICATION**

(21) Application number: 87303614.9

(22) Date of filing: 24.04.87

(51) Int. Cl.⁴: **A 61 F 13/20**

(30) Priority: 26.04.86 GB 8610285

(43) Date of publication of application:
11.11.87 Bulletin 87/46

(84) Designated Contracting States:
AT BE CH DE ES FR GB GR IT LI LU NL SE

(71) Applicant: **Caligen Foam Limited**
**Broad Oak**
**Accrington Lancashire (GB)**

(72) Inventor: **Ledger, John**
**c/o Caligen Foam Limited Broad Oak**
**Accrington Lancashire (GB)**

(74) Representative: **MacMaster, Alan Jeffrey et al**
**Swindell & Pearson 44 Friar Gate**
**Derby DE1 1DA (GB)**

(54) **Medical swabs.**

(57) A medical swab comprises an absorbent flexible body member formed from at least two layers of foamed plastics material adhered together by a discontinuous layer of an adhesive composition incorporating an X-ray detectable material. The foamed plastics material is preferably polyurethane foam, the X-ray detectable material consisting of or incorporating barium sulphate. There is also provided a chemical product comprising an adhesive incorporating an X-ray detectable material.

## Description

Medical Swabs

This invention relates to medical swabs for use in absorbing body fluids during medical and surgical operations.

Due to the nature of the situations in which medical swabs are employed they must not contain stiff or sharp sections which might damage living tissue. The swabs must also be absorbent to body fluids and toxicologically acceptable and are usually soft and flexible. Although such swabs are intended to be used in the course of operations and then removed and discarded, situations do arise where swabs are left inside patients after an operation has been completed. It is therefore desirable that such swabs should be X-ray detectable so that they may be detected and removed at a later date in the event they are not removed at the time of the operation.

It has previously been proposed to render medical swabs X-ray detectable by constructing them from materials incorporating threads incorporating barium sulphate. While this is effective in rendering the swabs X-ray detectable it has the disadvantage that they become unacceptably stiff. It is an object of the present invention to provide an X-ray detectable medical swab in which this disadvantage is obviated or mitigated.

According to one aspect of the invention there is provided a medical swab comprising an absorbent flexible body member incorporating a layer of X-ray detectable material. The layer is preferably discontinuous in order to minimise reduction in flexibility of the body member.

The layer of X-ray detectable material may comprise a coating on a surface of the body member. Alternatively the layer may be interposed between two parts of the body member and may advantageously comprise an adhesive serving to secure such parts together.

The absorbent flexible body member may comprise a woven or non-woven fabric but preferably comprises a foamed plastics material such as polyurethane foam. Alternatively the body member may comprise a combination of such materials.

The X-ray detectable material preferably consists of or incorporates barium sulphate.

According to a further aspect of the invention there is provided an adhesive composition incorporating an X-ray detectable material, such as barium sulphate. Such a composition may be employed in the production if a wide range of products for medical or other uses where adherence and X-ray detectability are required.

In one embodiment of the invention described by way of example only, a medical swab comprises a body member constructed from two layers of flexible hydrophilic polyurethane foam adhered together by way of an intermediate adhesive layer. The body member may be of any desired shape and size but is conveniently in the form of a cube of around 50 mm side or other shaped member of equivalent volume. The adhesive layer which secures the two parts of the body member together is preferably discontinuous and may consist, for example, of a series of dots or other discrete areas uniformly dispersed across the area of the surface to which it is applied. In this way the adhesive produces minimal interference with the flexibility of the body member.

The adhesive composition incorporates an X-ray detectable material. The preferred material is barium sulphate which is medically approved, easily obtained, inexpensive and not affected by body fluids or sterilization. Other X-ray detectable materials may however be employed such as organic salts derived from metals at the higher end of the periodic table. Compounds of gold, mercury, lead and bismuth may also be employed subject to satisfactorily meeting toxicological requirements. The adhesive material may otherwise be of any desired composition meeting the requirements for medical use. Suitable adhesive compositions include ethylene vinyl acetate copolymers such as Avabond HM 6647 sold by Avalon Chemical Company.

The quantity of barium sulphate or other X-ray detectable material incorporated in the adhesive composition may be varied dependent on the thickness of the adhesive layer. Typically between 10% and 60% by weight of the composition may comprise barium sulphate corresponding to layer thicknesses of 0.2 to 1.0 mm. Preferably the proportion of barium sulphate is in the region of 40% by weight of the adhesive layer.

The adhesive layer may typically be applied at a coverage rate of 50 to 250 grammes/$m^2$ of the body member surface at 40% barium sulphate loading. In general a coverage rate of around 120 grammes/$m^2$ would be appropriate.

The resultant product comprises two layers of soft flexible foam material adhered together to produce a medical swab meeting the various requirements referred to previously and which is X-ray detectable by virtue of incorporation of the barium sulphate or other X-ray opaque material. The discontinuous application of the adhesive layer minimises interference with the soft feel of the swab producing a medically acceptable product which maintains its flexibility despite incorporation of means for rendering it X-ray detectable.

Various modifications may be made without departing from the invention. For example while it is preferred that the body member be composed of polyurethane or other foamed plastics material it may consist of or incorporate woven or non-woven fabrics. In some cases the body member may consist of a single part only to which a coating of X-ray detectable material is applied, the coating preferably being discontinuous and confined to one surface of the article only and being non-adhesive. Alternatively the swab may consist of more than two parts or layers adhered together by adhesive layers at least one of which incorporates X-ray detectable material and which are preferably discontinuous.

Moreover while reference has been made herein primarily to medical swabs, the adhesive composition referred to constitutes an invention in its own right which may be employed in a wide range of medical or other situations where adhesion and X-ray detectability are required.

Whilst endeavouring in the foregoing specification to draw attention to those features of the invention believed to be of particular importance it should be understood that the Applicant claims protection in respect of any patentable feature or combination of features hereinbefore referred to whether or not particular emphasis has been placed thereon.

## Claims

1. A medical swab comprising an absorbent flexible body member characterised in that it incorporates a layer of X-ray detectable material.

2. A medical swab according to claim 1 characterised in that said body member comprises a fabric.

3. A medical swab according to claim 1 characterised in that said body member comprises a foamed plastics material.

4. A medical swab according to claim 3 characterised in that said body member comprises polyurethane foam.

5. A medical swab according to any preceding claim characterised in that said layer is discontinuous in order to minimise reduction in flexibility of the body member.

6. A medical swab according to any preceding claim characterised in that said layer comprises a coating on a surface of the body member.

7. A medical swab according to any of claims 1 to 5 characterised in that said layer is interposed between two parts of the body member.

8. A medical swab according to claim 7 characterised in that said layer comprises an adhesive serving to secure said parts together.

9. A medical swab comprising an absorbent flexible body member characterised in that the body member is formed from at least two layers of foamed plastics material adhered together by a discontinuous layer of an adhesive composition incorporating an X-ray detectable material.

10. A medical swab according to claim 8 or 9 characterised in that said adhesive is applied at a rate of 50 to 250 grammes/m$^2$ of the surface of said body member.

11. A medical swab according to claim 8, 9 or 10 characterised in that said X-ray detectable material comprises between 10% and 60% by weight of said adhesive composition.

12. A medical swab according to any preceding claim characterised in that the thickness of said layer is between 0.2 and 1.0 mm.

13. A medical swab according to any preceding claim characterised in that said X-ray detectable material consists of or incorporates barium sulphate.

14. A chemical product characterised in that it comprises an adhesive having an X-ray detectable material incorporated therein.

15. A product according to claim 14 characterised in that said adhesive is an ethylene vinyl acetate copolymer.

16. A product according to claim 14 or 15 characterised in that said X-ray detectable material comprises barium sulphate.